# EUROPEAN PATENT SPECIFICATION

(11) **EP 2 034 012 B1**
(45) Date of publication and mention of the grant of the patent: **06.10.2010**
(21) Application number: 08075222.3
(22) Date of filing: 27.03.2008
(51) Int. Cl.: C12N 7/00, C07K 14/08, C12Q 1/70, A61K 39/12

(54) **Vaccine against viral rabbit hemorrhagic disease**
Impfstoff gegen die Chinaseuche (RHD) bei Kaninchen
Vaccin contre la maladie hémorragique virale des lapins

(30) Priority: 05.09.2007 EP 07075759
(43) Date of publication of application: 11.03.2009
(73) Proprietor: Laboratorios Ovejero, S.A., 24005 León (ES)
(72) Inventor: Ovejero Guisasola, Juan Ignacio, 24005 Leon (ES)
(74) Representative: Temino Ceniceros, Ignacio

(56) References cited:
- BOGA J A ET AL: "Molecular cloning, sequencing and expression in Escherichia coli of the capsid protein gene from rabbit haemorrhagic disease virus (Spanish isolate AST/89)." THE JOURNAL OF GENERAL VIROLOGY SEP 1994, vol. 75 ( Pt 9), September 1994 (1994-09), pages 2409-2413, XP002485284 ISSN: 0022-1317
- PARRA F ET AL: "The amino terminal sequence of VP60 from rabbit hemorrhagic disease virus supports its putative subgenomic origin." VIRUS RESEARCH MAR 1993, vol. 27, no. 3, March 1993 (1993-03), pages 219-228, XP002485285 ISSN: 0168-1702
- GOULD A R ET AL: "The complete nucleotide sequence of rabbit haemorrhagic disease virus (Czech strain V351): use of the polymerase chain reaction to detect replication in Australian vertebrates and analysis of viral population sequence variation." VIRUS RESEARCH JAN 1997, vol. 47, no. 1, January 1997 (1997-01), pages 7-17, XP002485286 ISSN: 0168-1702
- DATABASE BIOSIS [Online] BIOSCIENCES INFORMATION SERVICE, PHILADELPHIA, PA, US; 1995, RASSCHAERT DENIS ET AL: "Sequence and genomic organization of a rabbit hemorrhagic disease virus isolated from a wild rabbit" XP002485287 Database accession no. PREV199598170920 & VIRUS GENES, vol. 9, no. 2, 1995, pages 121-132, ISSN: 0920-8569

## Description

The present invention relates to new strains of rabbit hemorrhagic disease Virus (RHDV), and to a vaccine for the protection of rabbits against rabbit hemorrhagic disease (RHD) containing these novel strains. The invention is also concerned with a process for the preparation of such a vaccine.

### BACKGROUND ART

In the People's Republic of China, outbreaks of an apparently new viral highly contagious and acute fatal disease in rabbits were observed in 1984. Rapid spread and early mortality were the main epidemiological characteristics. The characteristic pathological signs were haemorrhages in the respiratory system, liver, spleen, cardiac muscle, and occasionally in the kidneys.

Outbreaks of a disease showing clinical signs similar to those described China were also reported in several European, African and American countries. Reports are documented from Italy, Germany, France and Spain.

The causative agent of the disease is the virus denominated as Rabbit Hemorrhagic Disease Virus (RHDV). This virus was isolated for the first time and simultaneously in Spain (Parra, F. and Prieto, M., 1990, J. Virology, 64:4013-4015) and Germany (Ohlinger, V.F. et al., 1990, J. Virology, 64: 3331-3336). RHDV is 40 nm in size, non-enveloped with a single RNA chain as genomic material. Based on morphological data, it was initially classified as a picornavirus (Pu, B. et al. 1985, Chin. J. Vet. Med. 11:16-17) and parvovirus (Xu et al., 4th World Rabbit Congress, supra). However, based on the information corresponding to virion size, density, morphology and molecular structure, it has been definitively classified as a calicivirus.

The disease affects chiefly adult rabbits. Young sucking rabbits and fattening rabbits seem to be naturally protected.

The period of incubation is 2-3 days; the mortality rate is higher than 90 %. All rabbits can become infected with RHDV, but clinical signs are usually seen only in animals that are more than 40 to 50 days old. Typically, infected rabbits develop a fever and die suddenly within 12 to 36 hours of its onset. In some cases, the only symptoms are terminal squeals followed rapidly by collapse and death. Dullness, anorexia, congestion of the palpebral conjunctiva, or prostration may also be seen. Occasionally, animals develop neurologic signs, including incoordination, excitement, opisthotonos, and paddling. Some rabbits turn and flip quickly in their cages; this can resemble convulsions or mania. Respiratory symptoms, including dyspnea and a terminal, blood-stained, frothy nasal discharge, are sometimes seen. Approximately 5% to 10% of the animals experience a more chronic course with severe jaundice, lethargy, and weight loss, and death within one to two weeks.

After the first outbreak of the disease in China, in 1984, Chinese veterinary services could control the disease by extensive use of formaline inactivated vaccine produced from tissues of infected rabbits.

Different known strains of the virus has been identified: Isolate 'Spanish', Isolate'Czech', Isolate 'German', Isolate 'China', and Isolate 'lowa'. A Spanish isolate, AST/89, is known from J. Gen. Virol., Vol. 75, 1994; pages 2409-2413.

The morphology of the virus reveals that virions consist of a capsid, which is not enveloped, round with icosahedral symmetry. The isometric capsid has a diameter of 35-39 nm, which appear round to hexagonal in outline. The capsid surface structure reveals a regular pattern with distinctive features, wherein the capsomer arrangement is clearly visible, and the capsid shows 32 cup-shaped depressions.

The virus genome is not segmented and contains a single molecule of linear positive-sense, single-stranded RNA. The viral genome encodes structural proteins. Virions consist of 1 structural protein located in the capsid.

At the present time, several commercial vaccines exist, known as tissue vaccines, based on the use of extracts from organs of experimentally infected rabbits, in which the virulent agent is thermally or chemically inactivated. The efficiency of these preparations has been tested in laboratory (J.E. Peeters, D. Vandergheynst and R. Geeroms. 1992. Cuni-Sciences 7:101-106) and in the field, but they present serious inconvenients of antigenic variability and biological risk due to their mode of elaboration.

| **Vaccine** | **Laboratory** | **Composition per dose** |
|---|---|---|
| CYLAP® HVD | Fort Dodge Veterinaria | Inactivated Rabbit Haemorrhagic Disease Virus, ≥5.120 HAU. Dose: 1 ml |
| CUNIPRAVACRHD | Hipra | Inactivated Rabbit Haemorrhagic Disease Virus. Dose: 0,5 ml. |
| DERCUNIMIX | Merial Laboratorios, S.A. | Homologous Mixomatosis Virus, ≥ 10^{2,7} IDTC50; Inactivated Rabbit Haemorrhagic Disease Virus, ≥ 5 PD₉₀. Dose: 0,2 ml. |

Despite the above described research on the production of a vaccine against RHD there still exists a need to obtain an effective solution facilitating the production of a marker vaccine.

### SUMMARY OF THE INVENTION

The present invention relates to new strains of the virus causative of rabbit hemorrhagic disease (RHDV), and their use in the preparation of a vaccine against said disease.

Therefore, according to an aspect of the present invention, it is provided two new isolated strains of RHDV, which has been deposited at the European Collection of Cell Cultures (ECACC), CEPR, Porton Down, Salisbury, Wiltshire, SP4 0JG, UK in accordance with the conditions of the Budapest Treaty on the International Recognition of Deposit of Microorganisms for the Purposes of Patent Procedure (1977), and has been assigned deposit numbers RHDV-LO1 07060601 and RHDV-L02 07060602, deposit date 6^{th} June 2007.

Said new viruses has been designated by the inventors as LO1 and L02 respectively, for which reason throughout the present description the terms RHDV-LO1 07060601 and L01, and RHDV-L02 07060602 and L02 will be applied indiscriminately.

According to another aspect, the invention provides a vaccine against rabbit haemorrhagic disease that comprises the viruses with deposit number RHDV-LO1 0706060 and RHDV-LO2 07060602.

A further aspect of the present invention is related to the use of said RHDV strains in the preparation of vaccines for immunization against a viral infection caused by a RHDV, wherein the composition comprises an immunologically effective amount of the LO1 and LO2 strains and a physiologically acceptable carrier. Optionally, the composition includes an adjuvant. The RHD strains may be totally inactivated.

According to another aspect, the invention provides the use of the RHDV strains RHDV-LO1 07060601 and RHDV-LO2 07060602 in the preparation of a vaccine for immunization against a viral infection caused by a rabbit hemorrhagic disease virus.

According to another aspect, the invention provides the use of the inactivated rabbit hemorrhagic disease strains RHDV-LO1 07060601 and RHDV-L02 07060602 in the manufacture of a medicament for inducing immunity in a mammal susceptible to rabbit hemorrhagic disease virus infection

Yet in another aspect, the invention provides the use of a pure homogeneous immunologically effective mixture of the rabbit hemorrhagic disease virus strains RHDV-LO1 07060601 and RHDV-L02 07060602 in the preparation of a vaccine for immunization against a viral infection caused by a rabbit hemorrhagic disease virus.

In a further aspect, the invention provides a method for the preparation of the vaccine as defined above, which comprises:
a) mix an immunogenically effective amount of the rabbit hemorrhagic disease virus strains which are deposited at the European Collection of Cell Cultures (ECACC), CEPR, Porton Down, Salisbury, Wiltshire, SP4 0JG, UK with deposit Nos. RHDV-LO1 07060601 and RHDV-LO2 07060602;
b) optionally chemically inactivating the virus strains; and
c) adjuvantation of the final product.

### DETAILED DESCRIPTION OF THE INVENTION

### DEFINITIONS

The terms "vaccine" and "immunogenic composition" are defined herein in a broad sense to refer to any type of biological agent in an administrable form capable of stimulating an immune response in an animal inoculated with the vaccine. For purposes of this invention, the vaccine (immunogenic composition) typically includes the viral agent in a totally inactivated form. Vaccines in general may be based on either the virus itself or an immunogenic (antigenic) component of the virus.

Herein, the term "protection" when used in reference to a vaccine refers to the amelioration (either partial or complete) of any of the symptoms associated with the disease or condition in question. Thus, protection of rabbits from RHDV by the present vaccines generally results in a diminishing of virus shedding and/or one or more of the clinical symptoms associated with infection.

An "immunologically effective amount" refers to an amount of an immunogen sufficient to induce a detectable cellular or humoral immune response in an animal.

An "attenuated" virus refers to a virus that is either unable to colonize a host, unable to cause disease in a host or causes significantly reduced disease symptoms in a host. Attenuated viruses typically lack a genetic component involved in host colonization or pathogenicity.

A "protective immune response," as used herein, refers to a cellular or humoral immune response that prevents or delays infection or disease caused by a specified pathogen.

SEQ. ID NO: 1 is the whole nucleotide sequence of the RHD virus strain LO1 of the invention (position 30-4831). SEQ. ID NO: 2 is the whole nucleotide sequence of the RHD virus strain L02 of the invention (position 30-4831).

The sequences of RHD viruses L01 and L02 were compared with each other of the RHD viruses LO1 and L02 differ in 139 nucleotides (2,88%) within the determined sequences of the genomes (position 30-4831) resulting in 19 amino acid exchanges.

A comparison of the sequences of RHD viruses L01 and LO2 with the respective regions of published genome sequences of RHD viruses:
RHD virus isolate "China" (Acc. No. AY523410)
RHD virus isolate "Czech" (Acc. No. U54983)
RHD virus isolate "German" (Acc. No. NC_001543)
RHD virus isolate "lowa2000" (Acc. No. AF258618)
RHD virus isolate "Spain" (Acc. No. Z49271)

The comparison revealed that RHD virus strain LO2 shows the closest relationship to RHD virus strain LO1, see phylogenetic tree below.

Positions 30-4831 of the RHD viruses LO1, L02, isolate "China", isolate "Czech", isolate "German", isolate "lowa2000" and isolate "Spain" were aligned to each other to generate a phylogenetic tree. Phylogenies are rooted assuming a biological clock. The length of each pair of branches represents the distance between sequence pairs. The units at the bottom of the tree indicate the number of the substitution events.

Throughout this application, references to specific nucleotides are to nucleotides present on the coding strand of the nucleic acid. The following standard abbreviations are used throughout the specification to indicate specific nucleotides:
C=cytosine
A=adenosine
T=thymidine
G=guanosine

Also embraced by this invention are methods to detect and differentiate RHD virus strains L01 and L02 in finished vaccine by RT-PCR and restriction fragment length polymorphism analysis (RFLP-analysis).

The both two RHD virus strains L01 and L02 differ in about 2.9% of the nucleotide sequence. Some of these mismatches between virus strain L01 and LO2 alter recognition sequences of restriction endonucleases and can therefore be detected by RFLP-analysis.

A mismatch at position 2662 of the virus genome is detected generating a recognition sequence for restriction endonuclease Mval in strain LO1 and a mismatch at position 2115 of the virus genome generating a recognition sequence for restriction endonuclease Ecl136II in virus strain LO2.

In a fist step two internal fragments of the RHD virus genome are amplified by RT-PCR. These PCR amplicons harbour the aforementioned mismatches. Subsequently the PCR amplicons are digested with restriction endonuclease Mval or EcI136II and analysed by agarose gel electrophoresis. The specific digestion patterns of DNA fragments reveal if there are both RHD virus strains LO1 and L02 in finished vaccine. Thus the specific RT-PCR detect RHD virus RNA in the vaccine, whereas the digestion of the PCR amplicons differentiates between RHD strains L01 and L02.

By substantially homogenous it is meant that the isolate is not contaminated with more than 10 %, and preferably less than 5 % and most preferably less than 1 % of another RHDV isolate. In a preferred embodiment of the invention, the vaccine contains a pure homogenous LO1 isolate and LO2 isolate mixture i.e. devoid of any contamination with other.

The pure isolate does not suffer from the disadvantages of potential batch to batch variation between substrains and provides a product which is easier to ensure will meet consistent quality guidelines.

As it is known from bibliography no satisfactory growth conditions or sensitive cell substrates have been established for cultivating VHD-Virus *in vitro (*Bárcena, J., Morales, M., Vázquez, Boga, J. A., Parra, F.L., Lucientes. J., Pagès-Manté, A., Sánchez-Vizcaíno, J., Blasco, R., Torres, J.M., 2000. Horizontal transmissible protection against myxomatosis ans rabbit hemorrhagic disease by using a recombinant myxoma virus. J. Virology, 74 (3). 1114-1123*.;* O.I.E. Manual. Standard for diagnostic test and vaccines. 3rd Ed. 1996. Chapter 3.7.3*)*. Therefore, VHD virus replication is restricted to susceptible animals. Commercial vaccines against VHD are prepared from the livers of experimentally infected rabbits.

The vaccine of the invention may be prepared using conventional vaccine preparation techniques based on inactivated live virus, which are well-known and commonly employed by experts in the field, in such a manner that they contain an efficient viral dosage, which may be considered to be within the range of 128 and 512 HAU/dose.

A general description of the manufacture process of the vaccine of the invention is represented in the following flow chart:

| **Strain L01** | **Strain L02** |
|---|---|
| **INOCULUM** | **INOCULUM** |
| **(viral suspension)** | **(viral suspension)** |
| **↓** | **↓** |
| **Infection of rabbits** | **Infection of rabbits** |
| **↓** | **↓** |
| **HARVESTING** | **HARVESTING** |
| **↓** | **↓** |
| **PRODUCTION** | |
| **Antigen suspension (L01 strain)** | **Antigen suspension (L02 strain)** |
| **↓** | **↓** |
| **Bulk antigen (mixture 1:1 of L01 and L02 strains)** | |
| | **↓** |
| **Inactivation with BPL** | |
| | **↓** |
| **Adsorption on aluminium hydroxide** | |
| | **↓** |
| **Bottling** | |
| | **↓** |
| **Labelling and packing** | |
| **Product release** | **↓** |

According to an embodiment of the invention, the process comprises the chemical inactivation of the bulk antigen mixture of LO1 and LO2 strains. Preferably the chemical inactivation is carried out with β-propiolactone (BPL) or any other suitable chemical inactivating agent such as formaldehyde.

According to an embodiment of the invention, the vaccine viruses are present in a ratio between 1:10 and 10:1, preferably between 1:6 and 6:1. In a more preferred embodiment, the vaccine viruses are present in a ratio 1:1

The mixture ratio for each of the vaccine viruses was 128 HAU/dose or over for the RHD virus strain LO1 07060601; and 128 HAU/dose or over for the RHD virus strain LO2 07060602.

Preferably, the adjuvantation step is carried out by adding aluminium hydroxide, incomplete mineral oil or mixture thereof. Preferably the adjuvantation is carried out by adding aluminium hydroxide. In an embodiment of the invention, the aluminium hydroxide is used in a quantity equivalent to 2% of aluminium oxide. The aluminium hydroxide is added, preferably, when the final product is prepared, in the ratio of 25% of the final volume. That means, in a vaccinal dose of 1 ml, 5 mg of aluminium content.

In an embodiment of the invention, the inactivation step is carried out keeping the antigenic suspension to 4°C. Preferably, the inactivating agent is added in a 3‰ proportion. After addition of the inactivating agent, which is preferably β-propiolactone (BPL), the suspension is shaked until complete homogenisation. An haemagglutination test for checking the inactivation of the virus is performed before and after adding BPL.

In a preferred embodiment of the invention, the vaccine comprises a substantially homogeneous mixture of the RHDV strains RHDV-LO1 07060601 and RHDV-L02 07060602 which is not contaminated with more than 10 % of another RHDV isolate. Preferably the vaccine is not contaminated with more than 5 %, more preferably no more than 1 %. In a particularly preferred embodiment, the vaccine comprises a pure homogeneous mixture of the RHDV strains RHDV-LO1 07060601 and RHDV-LO2 07060602.

Generally, in the manufacture of vaccine of the invention, healthy looking rabbits of the breed "New Zealand White Rabbits" are used. Of course, other breeds could be used. They are selected and transported to accurate facilities, where they are housed in an isolated room, examined and kept under quarantine.

Both Strains of the Master Strain Virus (MStV) are isolated from sick rabbits. Each strain of the MStV are inoculated to 200 healthy rabbits in order to obtain the Master Seed Virus (MSV). At the end of this process, both strains (MSV-LO1 and MSV-LO2) are bottled, properly labelled and stored at a suitable temperature, which preferably is between -60°C and -100°C. These MSV-LO1 and MSV-LO2, are inoculated to rabbits and the Working Seed Virus (WSV) of each strain is obtained. When necessary, the WSV-LO1 and WSV-LO2 are inoculated to rabbits in order to produce the Inoculum to be used in the manufacture of the vaccine of the invention. After the obtention of the MStV, MSV, WSV and Inoculum, the presence of the virus is assessed by the tests of haemagglutination activity and virulence in rabbits. During all this process, the mortality rate of each strain has been kept constant.

According to an embodiment of the invention, the manufacturing process comprises the following steps:
i) defrost the Working Seed Virus (strain L01 and strain LO2);
ii) centrifuge and nebulize the WSV over the animals in two separated processes;
iii) collected the liver from the dead animals and store by freezing;
iv) grain the unfrozen livers in PBS pH 7,2 and kept in constant agitation at 4°C;
v) add β-Propiolactone (BPL) and shaking continuosly until total homogeneization;
vi) keep the suspension for one day at 37°C to get a total hydrolysis of BPL;
vii) add the adjuvant (aluminium hydroxide);
viii) keep the suspension in continuous shaking to guarantee the homogeneity of the batch until final process;

The mode of administration of the vaccine of the invention may be any suitable route which delivers an immunoprotective amount of the strain and other immunogenic component of the vaccine to the subject. However, the vaccine is preferably administered parenterally via the intramuscular or subcutaneous routes. More preferably the vaccine is administered subcutaneously. Other modes of administration may also be employed, where desired, thus examples of compositions of the invention include liquid preparations for orifice, e.g., oral, nasal, anal, vaginal, etc., administration such as suspensions, syrups or elixirs; and, preparations for parenteral, subcutaneous, intradermal, intramuscular or intravenous administration (e.g., injectable administration) such as sterile suspensions or emulsions. In such compositions the two RHD virus strains may be in admixture with a suitable carrier, diluent, or excipient such as sterile water, physiological saline, glucose or the like.

The appropriate immunoprotective and non-toxic dose of such vaccine can be determined readily by those skilled in the art, i.e., the appropriate immunoprotective and non-toxic amount of the strain of this invention contained in the vaccine of this invention may be in the range of the effective amounts of antigen. Preferably, the minimum recommended age for vaccination is approximately of 8 weeks of age, after weaning.

Of course, the administration can be repeated at suitable intervals if necessary. Preferably the vaccination schedule is the following:
First vaccination: 1 ml by subcutaneous route, after weaning.
Revaccination: yearly revaccination in breeding or female reposition animals (1 ml by subcutaneous route ).

Throughout the description and claims the word "comprise" and variations of the word, such as "comprising", is not intended to exclude other technical features, additives, components, or steps. For those skilled in the art, objects, advantages a d features of the invention will become apparent from the description and from practice of the invention. The following examples are provided by way of illustration.

### EXAMPLES

### Example 1. Manufacturing method.

### 1. Method to obtain the bulk antigen

The bulk antigen of each strain was separately produced. At the laboratory, 500 ml of the Working Seed Virus of each strain (LO1 and L02 strain), which are stored at -70°C, were defrosted within less than 15 minutes in warm water and centrifuged for 15 minutes. Only one virus strain was used at one time.

### 2. Nebulization technique and length of post-nebulization period.

The fluid containing virus was nebulized over the animals cages using a usual nebulizer commercially available for plant protection. The animals were nebulizated at the same time. All of them were placed in the same room. In case of infection with LO1 strain the death rate should exceed 70% of all animals within 3 days, using the L02 strain the death rate should exceed 60% within this timing. These accumulative death rates were part of the quality criteria: in case the death rates were below the target percentage, the whole process was terminated. Dead animals were immediately processed. All animals still alive after day 5 were slaughtered by cervical dislocation. Carcasses were placed in double bags and watertight and airtight containers, to be incinerated.

### 3. Organs collection technique

The carcasses were introduced in the processing area through the SAS system inside plastic bags. Each carcass was ventrally opened at the stomach area in routine autopsy method. All visible organs were carefuly examined to confirm RHD related diagnosis and specifically to exclude all animals, which might show symptoms/organ, changes not related to the infection with VHD. The liver was extracted under hygienic conditions and transferred into sterile PBS.

The organs were aseptically transferred to marked containers with the help of blunt scissors. The containers were filled up to 4 Kg liver, appropriate labelled (strain, batch, date, weight) and stored at -30 ° for a period of maximal 3 months.

### 4. Blending, inactivation and detoxification

The batch composition was calculated with the support of the calculation sheet PRODAR1.XLS. This finally results in: One batch of the vaccine of the invention was produced from no more than 12 Kg liver (from each strain) obtains from rabbits resulting in over 300.000 ml of the inactivated vaccine.

As it has been described, this vaccine was produced from rabbits livers. The harvested livers were blended in PBS pH 7.2 in a Silverson homogenizer at 18000 rpm, avoiding heating production during this phase (refrigerated double jacket). The antigenic suspension was kept to 4°C and in constant stirring and β - propiolactone (BPL) was added in a 3‰ proportion. The suspension is stirred until complete homogenisation.

### 5. Adjuvantation

Aluminium hydroxide was used as adjuvant, in a quantity equivalent to 2% of Aluminium oxide, which was systematically tested by adsorption in a Congo Red solution. The test wais read using a spectrophotometer with a 520 nm-length wave. The reading must be always lower than two in the batch that is considered as suitable.

The aluminium hydroxide was added when the final product was prepared, in the ratio of 25% of the final volume. That means, in a vaccinal dose of 1 ml, 5 mg of aluminium content. After inactivating the supernatant, the adjuvant was added to the container of the inactivated suspension (which has a stirring system) and kept for 24 hours at 37 °C.

### 6. Manufacturing and volume of a batch

As it was previously mentioned, two strains were used for the production of the vaccine. Each strain is used separately for infection of rabbits at the harvesting process. It is only before inactivation when both strains, L01 and LO2 were mixed in a volume of 1:1.

### 7. Bottling

The vaccine is stored in a stainless steel tank that has been previously sterilised and provided with a continuous stirring system, in order to keep the homogeneity of the batch for up to 24 hours at 4 °C. The product is transferred from the tank to the bottling area using sterile gas tube system. Bottling was carried out in a sterile environment, class 100 A, with 100 B background. Rubber stoppers and capsules were applied in the same area. The vials containing the final product were stored at 4 °C, until they were labelled and packed. Labelling and packing were carried out in a separated area, and only one batch of the final product and its conditioning material can enter this area each time. Final storage until marketing was carried out at 5 ± 3 °C.

### Example 2. Composition

| **NAME OF SUBSTANCES** | **QUANTITY PER DOSE (1 ml)** |
|---|---|
| **Active substances:** | |
| Organs suspension of Viral Haemorrhagic Disease LO1 Strain * | 36 mg |
| Organs suspension of Viral Haemorrhagic Disease LO2 Strain * | 36 mg |

| **Adjuvant:** | |
|---|---|
| Aluminium hydroxide (expressed as aluminium oxide) | 5 mg |

| **Diluent:** | |
|---|---|
| Sodium chloride | 4.8 mg |
| Potassium chloride | 0.12 mg |
| Disodium phosphate Dodecahydrate | 0.69 mg |
| Potassium dihydrogen phosphate | 0.12 mg |
| Calcium chloride | 0.08 mg |
| Water for injection qs to make | 1 ml |

| | |
|---|---|
| * Final suspension of both strains with minimum 128 HAU and maximum 512 HAU/dose. | |

### Example 3. Haemagglutination test

The main objective of this study is to check the validity of the haemagglutination test as a method to quantify the viral amount of the Viral Haemorrhagic Disease Virus (VHDV) in the vaccine of the invention.

The parameters to be examined were:
1. Inter-plate repetitivity of the assay.
2. Intra-plate repetitivity of the assay.
3. Repetitivity of the same assay in different dates.

### 1. Intra-plate analysis results

In this assay, 7 replicates of the same batch of vaccine of the invention were titrated in a 96-well microtiter plates, and this assay was repeated two more times in the same day with different batches of the vaccine. The serial dilutions from the sample ranged from 1/2 to 1/4096. The obtained results, according to S.O.P., are shown in the following table:

### Results of titration of the vaccine batches R001, R002 and R004. Intra-plate Analysis

| | **Haemagglutination titer of replicates** | | | | | | |
|---|---|---|---|---|---|---|---|
| **Batch** | 1 | 2 | 3 | 4 | 5 | 6 | 7 |
| **R001A** | 1/128 | 1/128 | 1/128 | 1/256 | 1/128 | 1/128 | 1/256 |
| **R002A** | 1/512 | 1/512 | 1/256 | 1/512 | 1/256 | 1/512 | 1/512 |
| **R004A** | 1/256 | 1/128 | 1/256 | 1/256 | 1/256 | 1/256 | 1/256 |

The obtained results indicate that there are no statistically significant differences between the different replicates of the same batch of the vaccine. At least, 5 out of 7 samples showed the same haemagglutinant activity in the same plate and the other values differ only in one well of haemagglutination.

Moreover, the batches used for the validation were chosen because different values of haemagglutination were observed in the final product. However, the intra-plate variability observed was similar for the 3 batches, independently from the final titer of the product.

### 2. Inter-plates analysis results

In this assay, the one batch of the vaccine of the invention (R004A), previously analyzed, was titrated the same day in 3 different plates (7 replicates per plate), with serial dilutions between 1/2 and 1/4096. The obtained results were the following:

### Results of titration of the vaccine batch R004. Analysis inter-plate

| | **Haemagglutination titer of replicates** | | | | | | |
|---|---|---|---|---|---|---|---|
| **Plate** | **1** | **2** | **3** | **4** | **5** | **6** | **7** |
| **A** | 1/256 | 1/128 | 1/256 | 1/256 | 1/256 | 1/256 | 1/256 |
| **B** | 1/256 | 1/256 | 1/256 | 1/256 | 1/256 | 1/256 | 1/256 |
| **C** | 1/256 | 1/256 | 1/128 | 1/256 | 1/256 | 1/256 | 1/256 |

The average was 1/256 in all plates with 1, 0 and 1 different values, respectively in each plate. So we can conclude that the method is repetitive and independent from the plate.

The same analysis was carried out with samples corresponding to the Master Seed Virus L01, stored at -70°C. In this case, 3 different vials of the MSV were analysed (7 replicates in 2 different plates) with serial dilutions ranged between 1/8 and 1/16384, because of the expected higher levels of haemagglutinant activity in this sample. Thus, this assay was valid for intra-and inter-plate variability analysis. The observed results are shown below:

### Results of titration of Master Seed Virus L01. Analysis inter-plate

| | **Haemagglutination titer of replicates** | | | | | | |
|---|---|---|---|---|---|---|---|
| **Vial** | **1** | **2** | **3** | **4** | **5** | **6** | **7** |
| **A** | 1/4096 | 1/4096 | 1/4096 | 1/8192 | 1/8192 | 1/4096 | 1/4096 |
| **A** | 1/4096 | 1/4096 | 1/8192 | 1/4096 | 1/8192 | 1/4096 | 1/4096 |
| **B** | 1/4096 | 1/4096 | 1/8192 | 1/8192 | 1/4096 | 1/4096 | 1/4096 |
| **B** | 1/8192 | 1/4096 | 1/4096 | 1/4096 | 1/4096 | 1/4096 | 1/4096 |
| **C** | 1/4096 | 1/4096 | 1/4096 | 1/4096 | 1/8192 | 1/4096 | 1/4096 |
| **C** | 1/8192 | 1/8192 | 1/4096 | 1/4096 | 1/4096 | 1/4096 | 1/4096 |

The average for the three vials was the same, 1/4096, with the value 1/8192 being represented twice in 4 plates and once in other 2 plates. These results show that the technique is also valid with high haemagglutinant activity.

### 3. Temporal analysis of haemagglutination test

To check the variability mainly due to the technician manipulations, the same batch of the vaccine of the invention (R004A) was titrated in 3 different days, with 7 replicates per plate and 3 plates each day. Every day, all necessary solutions were freshly prepared and the vaccine samples were stored at 4°C between the first and the last day of the experiment. The obtained results were the following:

### Results of titration of the vaccine batch R004A. Time Analysis.

| | **Haemagglutination titer of replicates** | | | | | | | |
|---|---|---|---|---|---|---|---|---|
| | | **1** | **3** | **3** | **4** | **5** | **6** | **7** |
| **Day 1** | **Plate A** | 1/256 | 1/256 | 1/128 | 1/256 | 1/256 | 1/256 | 1/128 |
| | **Plate B** | 1/128 | 1/256 | 1/128 | 1/256 | 1/256 | 1/256 | 1/256 |
| | **Plate C** | 1/256 | 1/256 | 1/128 | 1/128 | 1/256 | 1/256 | 1/256 |
| **Day 2** | **Plate A** | 1/128 | 1/256 | 1/256 | 1/256 | 1/256 | 1/256 | 1/256 |
| | **Plate B** | 1/256 | 1/128 | 1/128 | 1/256 | 1/256 | 1/256 | 1/256 |
| | **Plate C** | 1/256 | 1/256 | 1/256 | 1/128 | 1/256 | 1/256 | 1/256 |
| **Day 3** | **Plate A** | 1/256 | 1/256 | 1/256 | 1/256 | 1/128 | 1/128 | 1/256 |
| | **Plate B** | 1/256 | 1/256 | 1/256 | 1/128 | 1/256 | 1/128 | 1/256 |
| | **Plate C** | 1/128 | 1/256 | 1/256 | 1/256 | 1/256 | 1/256 | 1/256 |

The analysis of the first day shows an average of 1/256 and 15 out of 21 values obtained were identical, with the other 6 values being 1/128. Also no differences were observed between the 3 plates. The second and third day the averages were the same (1/256) with 4 and 5 values out of 21 other than the average (1/128).

The same experiment was carried out with the Master Seed Virus L01. One vial of MSV was fastly defrozen the first day of assay and the sample was analysed with 7 replicates in 3 different plates. Throughout the experiment (3 days), the sample was stored at 4°C to avoid activity loss due to freeze-defreeze. The results are shown below:

### Results of titration of Master Seed Virus L01. Time Analysis.

| **Haemagglutination titer of replicates** | | | | | | | | |
|---|---|---|---|---|---|---|---|---|
| | | **1** | **2** | **3** | **4** | **5** | **6** | **7** |
| **Day 1** | **Plate A** | 1/4096 | 1/8192 | 1/4096 | 1/4096 | 1/8192 | 1/4096 | 1/8192 |
| | **Plate B** | 1/4096 | 1/4096 | 1/4096 | 1/4096 | 1/4096 | 1/8192 | 1/8192 |
| | **Plate C** | 1/8192 | 1/8192 | 1/4096 | 1/4096 | 1/4096 | 1/4096 | 1/4096 |
| **Day 2** | **Plate A** | 1/4096 | 1/4096 | 1/409 | 1/8192 | 1/4096 | 1/4096 | 1/4096 |
| | **Plate B** | 1/4096 | 1/8192 | 1/8192 | 1/4096 | 1/4096 | 1/4096 | 1/4096 |
| | **Plate C** | 1/4096 | 1/4096 | 1/409 6 | 1/4096 | 1/4096 | 1/4096 | 1/4096 |
| **Day 3** | **Plate A** | 1/4096 | 1/4096 | 1/8192 | 1/4096 | 1/4096 | 1/4096 | 1/4096 |
| | **Plate B** | 1/4096 | 1/4096 | 1/2048 | 1/4096 | 1/4096 | 1/4096 | 1/4096 |
| | **Plate C** | 1/4096 | 1/4096 | 1/2048 | 1/4096 | 1/4096 | 1/4096 | 1/2048 |

These results show no differences in the average between plates of the same day, and between plates of different days. In all cases the average was 1/4096. However, slight differences were observed in the results of the third day, where we could observe some replicates (3) with haemagglutination titers of 1/2048, values not observed in the first and second day of experiments. This result is in agreement with some light haemagglutinant activity loss due to storage at 4°C.

The results shown beside indicate that the haemagglutination test of human red-blood-cells by VHDV, according to our internal protocol, is valid to determine the viral activity through the manufacturing process of the vaccine of the invention.

No significant differences of haemagglutinant activity were observed in intra-plate, inter-plate and temporal assays, with samples of the vaccine of the invention or the Master Seed Virus L01. Moreover, the larger differences observed in the values of haemagglutination in the replicates of the same sample were only 1 serial dilution (except samples of 3rd day of time analysis of MSV). Might be a titration method with serial dilutions in base 1,5 instead of base 2 could be more precisse in determining the exact titer of a sample, but this data do not affect the validity of the current method.

### Example 4.. Immunogenicity test

The aim of this test is to check the immunogenicity of the vaccine of the invention.

Two groups of 10 animals (4 months old and randomly selected male and not breeding females) were vaccinated with one dose (1 ml) of the vaccine of the invention, by subcutaneous route, while other 2 groups of 10 animals were kept as control, without been inoculated.

Serological status before vaccination, 7 and 14 days after vaccination was checked. Animals were controlled for 14 days after vaccination. The test were performed using production batches with the minimum allowed antigenic charge (128 and 256 HA units, respectively).

### Haemagglutination inhibiting antibodies titres

The titres of control animals remained below 1/20 HAU, while the titter of vaccinated animals reached 1/320. Some animals even reached 1/640. Therefore, vaccinated animals increased their titters 4-5 times in response to vaccination.

The immunogenicity of one dose of the vaccine of the invention has been demonstrated, as the titter did rise 5 - 6 times during observation period (14 days).

### Protection level

These studies were carried out to determine under controlled laboratory conditions the protection level due to the inactivated vaccine of the invention.

### Study A:

Groups of rabbits vaccinated and non-vaccinated (control group) of the previous Immunogenicity test were challenge 15 days after vaccination with 0.5 ml intramuscularly dose containing no less than 1/5120 HAU.

Mortality was checked during the following 3 days.

All vaccinated animals survived the observation period while 8 control rabbits died during the second and third day after challenge. Only 2 control rabbits survived the challenge.

Conclusions: Animals vaccinated with the vaccine of the invention and challenged after 2 weeks are protected against RHD.

### Study B:

20 rabbits were selected according to their serological status within a group of 16 weeks old animals that had been inoculated with different dilutions of the vaccine at the 11th week. The rabbits were divided in five groups with 4 rabbits each, according to their serological status: 1/20, 1/40, 1/80, 1/160 and 1/320. 14 days after vaccination all animals were challenged: 0.5 ml of a field virus suspension, containing no less than 5120 HAU by intramuscular route. A study of the serological status before and after the challenge was carried out in all animals.

Mortality was daily checked after the challenge. The test was performed using a production batch with the minimum allowed antigenic charge (128 HA units).

The animals with initial titres of 1/20 and 1/40 died in the 4-7 days after the challenge. All animals with initial titter of 1/80 or higher survived the challenge. Seven days after challenge, their antibody rate reached titres of 1/320 (7 rabbits), 1/640 (4 rabbits) and one animal showed s titter of 1/1280.

Conclusions: Titter of 1/80 HAU protects rabbits from VHD infection. Therefore the titter of 1/80 is considered a protective level.

### Example 5. Determination of the vaccinal dose

This trial was aimed to determine the vaccinal dose of the vaccine of the invention for adult and weaning rabbits under laboratory conditions.

21 animals of each category (weaning -8 weeks old- and adult rabbits -3 months old-), random male/female, were divided in 7 groups of 3 animals each. 6 groups of each category were vaccinated with different dilutions of the vaccine. One group of each category (three animals) remained unvaccinated, as control group.

All groups were intramuscularly challenged with 0.5 ml, no less than 5120 HUA of the field virus. Adult rabbits were challenged 7 days after vaccination, while weaning rabbits were challenged 30 days after vaccination.

Until 72 hours after the challenge, general condition and mortality were supervised two times a day and rectal temperature was checked 24, 36, 48 and 72 hours after challenge. In adults rabbits serology was performed before vaccination, 7 days after vaccination and 7 days after challenge. In weaning rabbits serology was performed at vaccination, 7 days after, 30 days after vaccination (just before the challenge) and 7 days after challenge.

A vaccination batch with the minimum allowed antigenic charge (128 HA units) was used.

### Results:

Clinical status: After challenge, the general status of most vaccinated animals was normal. Control animals were depressed until they died.
Temperature: the temperature of vaccinated were within the range. Control animals had slightly higher temperatures.
Mortality: Only 1 weaning rabbit of the 21 vaccinated animals died during the study, while all control animals did.
Serological studies: 7 days after vaccination young rabbits had lower values than adults had. However the titter were almost similar 30 after vaccination at the end of the study. After challenge, antibodies titres of all adult and weaning vaccinated rabbits increased 1 or 2 folds if the titters was 1/160 or below before challenge.

Conclusion: 1/32 dilutions of one dose of 128 HAU (equivalent to 4 HAU) protect rabbits from dying from VHD in almost cases. The fact that there is no increase in titres in animals vaccinated with 1 or ½ dose (equivalent to 128 and 64, respectively) after been challenged reveals that titres of 1/160 or higher do protect rabbits from infection.

### Example 6. Immunity appearance

This study was carried out to determine under controlled laboratory conditions the appearance of the immunity due to the vaccine of the invention.

Two groups of adult animals (with 35 and 5 rabbits each) were used. The biggest group was vaccinated with 1 ml of the product, while the other was kept as the control group. 7 days after vaccination both groups were challenged with 0.5 ml of the field virus by intramuscular route (no less than 5120 HUA) and observed for 28 days after the challenge. Serum samples were taken daily from vaccination till challenge. The test were performed using a production batch with the minimum allowed antigenic charge (128 HA units).

### Results:

There was a seroconversion from the 4th until the 7th day after vaccination, starting with 1/40 to 1/80. The titres increased during the following days, becoming stabilised (1/320) at about the 7th days post-vaccination. Control animals remained below 1/20 and after the challenge all control animals died. No vaccinated animal died after challenge.

Conclusion: Seroconversion begins 4 days after the vaccination and stabilised after 7 days. Protective titres are reached in the 4th day after vaccination.

### Example 7. Immunity length

This study was made to check the immunity length after vaccination to rabbits, under field conditions. Kinetics of haemagglutination inhibiting antibodies were also carried out.

50 rabbits (4 months old) were vaccinated with 1 ml of the vaccine of the invention containing 128 Haemagglutinating units after checking their seronegativity for the VHD Virus. A serological study was carried out during 12 months, in the days 3, 4, 5, 6, 7, 14, 21, 28, 60, 120, 180, 240, 300 and 360 after vaccination. The test were performed using the production batch 01/009, with the minimum allowed antigenic charge (128 HA units).

During the observation period, the animals had serological titres of at least 1/80 and higher. From the 8th month after vaccination, the titre started to decrease, but always kept above 1/80.

Conclusion: Serological titres remained at a level to stimulate immune response 12 months after vaccination.

### Example 8. Revaccination

The study was carried out to determine the immunity after revaccination under field conditions.

20 adult females (14 - 15 months old) were used in this trial. The animals had been vaccinated in the farm one year before this test, in the field trial "Immunity length". Before starting the study, blood samples were taken from every animal in order to determine their haemagglutination inhibiting antibody titre before re-vacination.

The rabbits were revaccinated with 1 ml of the vaccine of the invention. 1 ml of the vaccine was inoculated on the left flank of the rabbit, after superficial disinfection of the skin with ethanol 70°. There are no control animals.

Haemagglutination inhibiting antibody titres were determined between day 1 and 21 after vaccination. For 21 days after the product administration the animals were controlled: the haemagglutination inhibiting antibodies titres were checked in the days 1, 2, 7,14 and 21 after revaccination, animals were clinically observed two times a day and mortality was controlled until the end of the study. The test were performed using a production batch with 128 HA units.

More than 50% of the re-vaccinated animals reached titres of 1/320 at the 21st day, while the rest of animals values were 1/1280 and 1/640. This proves the clear existence of booster effect. The protective level (1/80) was reached in all animals.

### Conclusion:

As the protective level (1/80) was reached in all animals and the increase in haemagglutinant inhibition titres shows the existence of booster effect, we can conclude that the immunity after revaccination is appropriate. As no local or general reactions have appeared, the safety of revaccination has also been checked.

### Example 9. Detection of RHD virus strains LO1 and LO2 in finished vaccine.

The aim of the study was to develop a RT-PCR assay and restriction fragment length polymorphism analysis (RFLP-analysis) which can detect and differentiate RHD virus strains LO1 and LO2 in finished vaccine. by RT-PCR.

An analysis of the determined sequences revealed that both strains are quite homologous to each other. Nevertheless RHD virus strains L01 and L02 show some mismatches which generate or delete recognition sequences of restriction endonudeases.

Two suitable mismatches were identified which generate recognition sequences of restriction endonucleases Mval and EcI135II. An T to C exchange at position 2662 generates a recognition sequence for Mval in RHD virus strain L01. An A to G exchange at position 2115 generates a recognition sequence for EcI136II in RHD virus strain L02. Thus RHD virus strain LO1 shows a recognition sequence for Mval at position 2662 but no recognition sequence for EcI136II at position 2115 whereas RHD virus strain LO2 shows a recognition sequence for EcI136II at position 2115 but no recognition sequence for Mval at position 2662. These differences in the genome sequences of RHD virus strains L01 and LO2 can be detected by amplifying suitable fragments of the virus genome by RT-PCR that followed a digestion of the RT-PCR products with the appropriate restriction endonuclease.

Primers for two quality control RT-PCR (PCR RHD-QC-1 and PCR RHD-QC-2) were designed which amplify internal fragments of the RHD virus genome, each harbouring one of the two mismatches at position 2115 or 2662.

Thus, SEQ.ID.NO: 3 (aacaacatcc tgccgttcca c ) and SEQ.ID.NO:4 (aggttggggt cggtgaacac gt) are the upstream and downstream primers respectively of the PCR RHD-QC-1.

SEQ.ID.NO: 5 corresponds to the sequence of the PCR amplicon of PCR RHD-QC-1 of RHD virus strain LO1. SEQ.ID.NO: 6 corresponds to the sequence of the PCR amplicon of PCR RHD-QC-1 of RHD virus strain L02.

The PCR RHD-QC-1 amplifies a 359bp internal fragment of the RHD virus genome (position 2500-2858) harbouring the mismatch at position 2662.

SEQ.ID.NO: 7 (tcctgctaca gcaaggatat g) and SEQ.ID.NO:8 (tactccacac acccttcaat c) are the upstream and downstream primers respectively of the PCR RHD-QC-2.

SEQ.ID.NO. 9 corresponds to the sequence of the PCR amplicon of PCR RHD-QC-2 of RHD virus strain LO1. SEQ.ID.NO. 10 corresponds to the sequence of the PCR amplicon of PCR RHD-QC-2 of RHD virus strain L02.

PCR RHD-QC-2 amplifies an 371 bp internal fragment of the RHD virus genome (position 1981-2351) harbouring the mismatch at position 2115.

Virus stocks were stored in aliquots (150 µl) at -70°C. Vaccine samples were centrifugated to remove aluminium hydroxide. Vaccine samples were stored in aliquots (300µl) at -70°C.

Purification of viral RNA from both RHD virus strains and from vaccine was performed using the QIAamp viral RNA Mini kit (Qiagen). All subsequent experiments were performed with purified viral RNA.

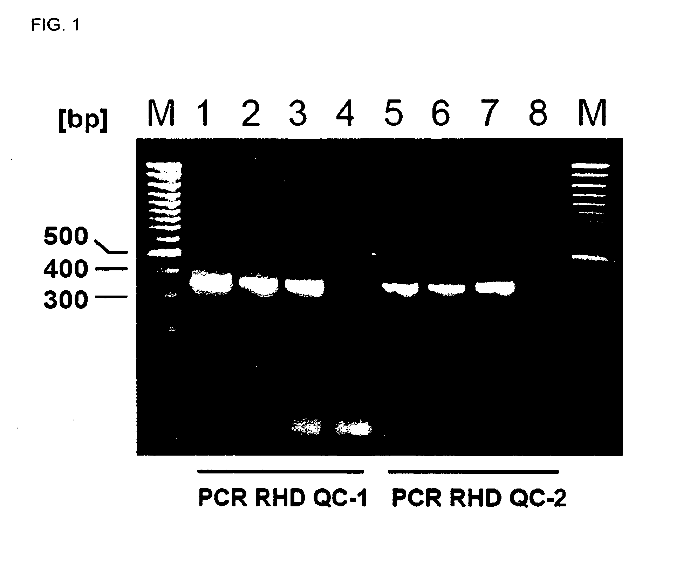

The conditions for the QC-PCRs RHD-QC-1 and RHD-QC-2 were established and optimized for the use of OneStep RT-PCR Kit (Qiagen). Qiagens OneStep RT-PCT Kit allows a sensitive one-step RT-PCT with viral RNA as template. The RT-PCR setup combines gene-specific primers and all reaction components needed for cDNA synthesis and PCR in one tube. Nothing needs to be added once the reaction has been started. Using the optimized RT-PCT we were able to amplify RHD viruses LO1 (Fig. 1 lanes 1 and 5) and L02 (Fig 1 lanes 2 and 6) reproducibly both from virus stocks and from vaccine (Fig. 1 lanes 3 and 7).

QC-PCRs RHD-QC-1 and RHD-QC-2 are specific for RHD viruses. Therefore a specific RT-PCR amplicon in a RT-PCR assay using RNA as template which was purified from vaccine demonstrates that vaccine contains either RHD virus strain L01 or RHD virus strain L02 or both RHD virus strains L01 and L02.

To investigate whether vaccine contains just one of the RHD virus strains of both of them, we used RFLP-analysis.

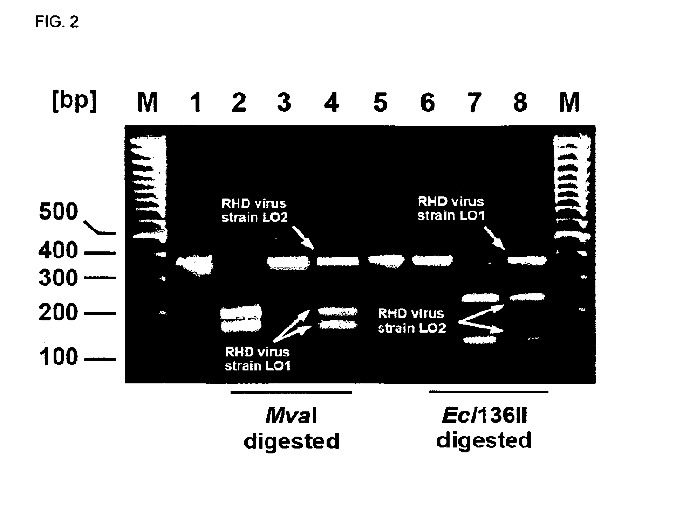

PCR RHD-QC-1 amplifies an 359bp internal fragment of the RHD virus genome, which contains a Mval recognition sequence in RHD virus strain LO1 but not in strain LO2. Thus a digestion of the PCR amplicon RHD-QC-1 with the restriction endonuclease Mval produces either two DNA fragments (196bp and 163bp) if the genome of RHD virus LO1 was the template for the RT-PCR (Fig.2 lane 2) or just one DNA fragment (359bp) if the genome of RHD virus L02 was the template (Fig.2 lane 3). In case of Mval digestion of the RT-PCT-QC-1 amplicon produces three specific DNA fragments of 359bp, 196bp and 163bp then the sample contains both RHD virus strains L01 and LO2 (Fig.2 lane 4).

To verify the results of QC-PCR RHD-QC-1 a RFLP-analysis of QC-PCR RHD-QC-2 was established.

PCR RHD-QC-2 amplifies an 371 bp internal fragment of the RHD virus genome, which contains a EcI136II recognition sequence in RHD virus strain LO2 but not in strain LO1. Thus a digestion of the PCR amplicon RHD-QC-2 with the restriction endonuclease EcI136II produces either two DNA fragments (234bp and 137bp) if the genome of RHD virus strain LO2 was the template for the RT-PCR (Fig.2 lane 7) or just one DNA fragment (371 bp) if the genome of RHD virus strain L01 was the template (Fig.2 lane 6). In case of EcI136II digestion of the RT-PCT-QC-2 amplicon produces three specific DNA fragments of 371 bp, 234bp and 137bp then the sample contains both RHD virus strains LO1 and LO2 (Fig.2 lane 8).

As shown in Fig.2 the developed RT-PCR assay detects and differentiates RHD virus strains LO1 and LO2 in vaccine (lanes 4 and 8) as well as in the virus stocks (lanes 2,3,6 and 7).

## Claims

1. A vaccine against rabbit hemorrhagic disease virus which comprises a immunologically effective amount of the rabbit hemorrhagic disease virus strains which are deposited at the European Collection of Cell Cultures (ECACC), CEPR, Porton Down, Salisbury, Wiltshire, SP4 0JG, UK with deposit Nos. RHDV-LO1 07060601 and RHDV-L02 07060602, optionally together with a physiologically acceptable carrier.

2. The vaccine according to claim 1 which includes an adjuvant selected from aluminum hydroxide, incomplete mineral oil and mixtures thereof.

3. The vaccine according to any of claims 1-2, **characterized in that** the two strains of rabbit hemorrhagic disease virus are chemically inactivated.

4. The vaccine according to claim 3, wherein the chemical inactivation is carried out by treatment of the virus with a chemical inactivating agent selected from [beta]-propiolactone and formaldehyde.

5. The vaccine according to any of claims 1-4, **characterized in that** the mixture ratio for each of the vaccine viruses was 128 HAU/dose or over for the RHD virus strain L01 07060601; and 128 HAU/dose or over for the RHD virus strain L02 07060602."

6. The vaccine according to any of claims 1-5, **characterized in that** the vaccine viruses are present in a ratio between 1:10 and 10:1.

7. The vaccine according to any of claims 1-6, wherein the vaccine viruses are present in a ratio between 1:6 and 6:1.

8. The vaccine according to any of claims 1-7, wherein the vaccine viruses are present in a ratio 1:1.

9. The vaccine according to any of claims 1-8, **characterized in that** it comprises a substantially homogenous mixture of the rabbit hemorrhagic disease virus strains RHDV-LO1 07060601 and RHDV-L02 07060602 which is not contaminated with more than 10 % of another rabbit hemorrhagic disease virus isolate.

10. The vaccine according to any of claims 1-9, **characterized in that** it comprises a pure homogenous mixture of the rabbit hemorrhagic disease virus strains RHDV-LO1 07060601 and RHDV-L02 07060602.

11. The vaccine according to any of claims 1-10, **characterized in that** it contains an efficient viral dosage within the range of 128 and 512 HAU/dose

12. Use of a rabbit hemorrhagic disease virus strain having the nucleotide sequence depicted in SEQ.ID.NO.1 in the preparation of a vaccine for immunization against a viral infection caused by a rabbit hemorrhagic disease virus.

13. Use of a rabbit hemorrhagic disease virus strain having the nucleotide sequence depicted in SEQ.ID.NO.2 in the preparation of a vaccine for immunization against a viral infection caused by a rabbit hemorrhagic disease virus.

14. Use of an inactivated rabbit hemorrhagic disease virus strain having the nucleotide sequence depicted in SEQ.ID.NO.1 and an inactivated rabbit hemorrhagic disease virus strain having the nucleotide sequence depicted in SEQ.ID.NO.2 in the manufacture of a medicament for inducing immunity in a mammal susceptible to rabbit hemorrhagic disease virus infection.

15. Use of a pure homogeneous immunologically effective mixture of the rabbit hemorrhagic disease virus strain having the nucleotide sequence depicted in SEQ.ID.NO.1 and the inactivated rabbit hemorrhagic disease virus strain having the nucleotide sequence depicted in SEQ.ID.NO.2 in the preparation of a vaccine for immunization against a viral infection caused by a rabbit hemorrhagic disease virus.

16. A method for the preparation of the vaccine according to any of claims 1-11, which comprises:
a) mix an immunogenically effective amount of the rabbit hemorrhagic disease virus strains which are deposited at the European Collection of Cell Cultures (ECACC), CEPR, Porton Down, Salisbury, Wiltshire, SP4 0JG, UK with deposit Nos. RHDV-LO1 07060601 and RHDV-L02 07060602;
b) optionally chemically inactivating the virus strains; and
c) adjuvantation of the final product.

17. The method according to claim 16, which comprises the chemically inactivation of the virus strains.

18. The method according to claim 17, wherein the chemical inactivation is carried out with by treatment of the virus with a chemical inactivating agent selected from [beta]-propiolactone and formaldehyde.

19. A rabbit hemorrhagic disease virus strain which is deposited at the European Collection of Cell Cultures (ECACC), CEPR, Porton Down, Salisbury, Wiltshire, SP4 0JG, UK with deposit No. RHDV-LO1 07060601.

20. A rabbit hemorrhagic disease virus strain which is deposited at the European Collection of Cell Cultures (ECACC), CEPR, Porton Down, Salisbury, Wiltshire, SP4 0JG, UK with deposit No. RHDV-L02 07060602.

21. A method for the detection and differentiation of attenuated RHD virus strains in a vaccine having the nucleotide sequence depicted in SEQ ID NO. 1 and a nucleotide sequence depicted in SEQ ID NO. 2, which comprises:
a) subjecting the genomic DNA of a sample of RHD vaccine to sequence analysis by RT-PCR and confirm that the genomic DNA of said sample RHD vaccine contains specific 359bp and 371 bp PCR-products;
b) digest the PCR product with the restriction endonucleases Mval and EcI136II;
c) confirm by restriction fragment length polymorphism analysis the presence of a recognition sequence for Mval at position 2662 but no recognition sequence for Ec1136II at position 2115 for the RHD virus strain LO1 having a nucleotide sequence depicted in SEQ ID NO:1, and a recognition sequence for EcI136II at position 2115 but no recognition sequence for Mval at position 2662 for the RHD virus strain LO2 having a nucleotide sequence depicted in SEQ ID NO:2.

22. The method according to claim 21, wherein the RT-PCR step a) is carried out using the following primers:
i) a pair of primers of SEQ.ID.NO. 3 and SEQ.ID.NO.4 related to the upstream and downstream primers respectively for the L01 detection;
ii) a pair of primers of SEQ.ID.NO. 7 and SEQ.ID.NO.8 are the upstream and downstream primers respectively for the L02 detection.

23. The method according to any of claims 21-22, wherein the pair of primers i) and ii) amplify two internal fragments of the RHD virus genome, each harbouring one of the two mismatches between L01 genome sequence and L02 genome sequence at position 2115 and 2662 respectively

## Patentansprüche

1. Eine Impfung gegen das Virus der hämorrhagischen Kaninchenkrankheit (RHSV), die eine immunologisch wirksame Menge Erregerstämme von RHSV umfasst, die in der Europäischen Zellkultursammlung (European Collection of Cell Cultures / ECACC), CEPR, Porton Down, Salisbury, Wiltshire, SP4 0JG, Großbritannien UK unter den Hinterlegungs-Nr. RHDV-LO1 07060601 und RHDV-LO2 07060602 hinterlegt sind, wahlweise zusammen mit einem physiologisch akzeptablem Trägerstoff.

2. Die Impfung gemäß Patentanspruch 1, die ein ausgewähltes Adjuvans aus Aluminiumhydroxid, inkomplettes Mineralöl und Mischungen davon enthält.

3. Die Impfung gemäß der Patentansprüche 1-2, **dadurch gekennzeichnet, dass** die zwei Virenstämme der hämorrhagischen Kaninchenkrankheit chemisch inaktiviert sind.

4. Die Impfung gemäß Patentanspruch 3, wobei die chemische Inaktivierung durch eine Behandlung des Virus mit einem aus [beta]-Propiolakton und Formaldehyd gewählten chemischen Inaktivierungsmittel erfolgt.

5. Die Impfung gemäß der Patentansprüche 1-4, **dadurch gekennzeichnet, dass** das Mischungsverhältnis für jedes Impfvirus 128 Hämagglutinin-Einheiten (HAU)/Dosis oder höher für den RHD-Virus-Stamm LO1 07060601 und 128 Hämagglutinin-Einheiten/Dosis oder höher für den RHD-Virus-Stamm LO2 07060602 beträgt.

6. Die Impfung gemäß der Patentansprüche 1-5, **dadurch gekennzeichnet, dass** die Impfviren in einem Mischungsverhältnis von 1:10 bis 10:1 auftreten.

7. Die Impfung gemäß der Patentansprüche 1-6, wobei die Impfviren in einem Mischungsverhältnis von 1:6 bis 6:1 auftreten.

8. Die Impfung gemäß der Patentansprüche 1-7, wobei die Impfviren in einem Mischungsverhältnis von 1:1 auftreten.

9. Die Impfung gemäß der Patentansprüche 1-8, **dadurch gekennzeichnet, dass** sie eine im Wesentlichen homogene Mischung der Virenstämme RHDV-LO1 07060601 und RHDV-L02 07060602 der hämorrhagischen Kaninchenkrankheit aufweisen, die mit höchstens 10% eines anderen Virus-Isolats der hämorrhagischen Kaninchenkrankheit kontaminiert ist.

10. Die Impfung gemäß der Patentansprüche 1-9, **dadurch gekennzeichnet, dass** sie eine reine homogene Mischung der Virenstämme RHDV-LO1 07060601 und RHDV-L02 07060602 der hämorrhagischen Kaninchenkrankheit aufweist.

11. Die Impfung gemäß der Patentansprüche 1-10, **dadurch gekennzeichnet, dass** sie eine wirksame Virusdosierung im Bereich von 128 bis 512 Hämagglutinin-Einheiten/Dosis aufweist.

12. Verwendung eines Virus-Stammes der hämorrhagischen Kaninchenkrankheit, der die in SEQ.ID.NO.1 beschriebene Nukleotid-Sequenz im Präparat einer Impfung zur Immunisierung gegen eine vom Virus der hämorrhagischen Kaninchenkrankheit verursachte Virusinfektion hat.

13. Verwendung eines Virus-Stammes der hämorrhagischen Kaninchenkrankheit, der die in SEQ.ID.NO.2 beschriebene Nukleotid-Sequenz im Präparat einer Impfung zur Immunisierung gegen eine vom Virus der hämorrhagischen Kaninchenkrankheit verursachte Virusinfektion hat.

14. Verwendung eines inaktivierten Virus-Stammes der hämorrhagischen Kaninchenkrankheit, der die in SEQ.ID NO.1 beschriebene Nukleotid-Sequenz hat und eines inaktivierten Vurus-Stammes der hämorrhagischen Kaninchenkrankheit, der die in SEQ.ID.NO.2 beschriebene Nukleotid-Sequenz hat, in der Herstellung eines Arzneimittels zur Herbeiführung der Immunität bei einem für die Infektion durch das Virus der hämorrhagischen Kaninchenkrankheit anfälligen Säugetier.

15. Verwendung einer reinen, homogenen, immunologisch wirksamen Mischung des Virus-Stammes der hämorrhagischen Kaninchenkrankheit, der die in SEQ.ID.NO.1 beschriebene Nukleotid-Sequenz hat und eines inaktivierten Virus-Stammens der hämorrhagischen Kaninchenkrankheit, der die in SEQT5.NO.2 beschriebene Nukleotid-Sequenz im Präparat einer Impfung zur Immunisierung gegen eine vom Virus der hämorrhagischen Kaninchenkrankheit verursachte virale Infektion hat.

16. Eine Methode für das Präparat der Impfung gemäß der Patentansprüche 1-11, die Folgendes umfasst:
a) Mischung einer immunogen wirksamen Menge der Virus-Stämme der hämorrhagischen Kaninchenkrankheit, die in der Europäischen Zellkultursammlung (European Collection of Cell Cultures / ECACC), CEPR, Porton Down, Salisbury, Wiltshire, SP4 0JG, Großbritannien unter der Hinterlegungs-Nr. RHDV-LO1 07060601 und RHDV-L02 07060602 hinterlegt sind;
b) wunschweise chemische Inaktivierung der Virus-Stämme und
c) Adjuvantierung des Endprodukts.

17. Die Methode gemäß Patentanspruch 16, die die chemische Inaktivierung der Virus-Stämme umfasst.

18. Die Methode gemäß Patentanspruch 17, wobei die chemische Inaktivierung durch eine Behandlung des Virus mit einem aus [beta]-Propiolakton und Formaldehyd gewählten chemischen Inaktivierungsmittel erfolgt.

19. Ein Virus-Stamm der hämorrhagischen Kaninchenkrankheit, der unter der Hinterlegungs-Nr, RHDV-LO1 07060601 in der Europäischen Zellkultursammlung (European Collection of Cell Cultures / ECACC), CEPR, Porton Down, Salisbury, Wiltshire, SP4 0JG, Großbritannien, hinterlegt ist.

20. Ein Virus-Stamm der hämorrhagischen Kaninchenkrankheit, der unter der Hinterlegungs-Nr. RHDV-L02 07060602 in der Europäischen Zellkultursammlung (European Collection of Cell Cultures / ECACC), CEPR, Porton Down, Salisbury, Wiltshire, SP4 0JG, Großbritannien, hinterlegt ist.

21. Eine Methode zum Nachweis und zur Differenzierung von abgeschwächten RHD-Virus-Stämmen in einer Impfung, die eine in SEQ.ID.NO.1 beschriebene Nukleotid-Sequenz und eine in SEQ.ID.NO.2 beschriebene Nukleotid-Sequenz haben, was Folgendes umfasst:
a) Sequenzanalyse der genomischen DNA einer Probe der RHD-Impfung durch RT-PCR und Bestätigung, dass die genomische DNA dieser Probe der RHD-Impfung ein spezifisches 359bp und 371 bp PCR-Produkt enthält.
b) Auslaugen des PCR-Produkts mit den Restriktionsendonukleasen Mval und Ec113611;
c) Durch Restriktionsfragment-Längen-Polymorphismen-Analyse Bestätigung der Präsenz einer Erkennungssequenz für Mval in Position 2662, aber keiner Erkennungssequenz für Ec113611 in Position 2115 für den RHD-Virus-Stamm LO1, der eine in SEQ.ID-NO. 1 beschriebene Nukleotid-Sequenz hat und eine Erkennungssequenz Ec113611 in Position 2115, aber keine Erkennungssequenz für Mval in Position 2662 für den RHD-Virus-Stamm LO2, der eine in SEQ.ID.NO. 2 beschriebene Nukleotid-Sequenz hat.

22. Die Methode gemäß Patenanspruch 21, wobei der RT-PCR-Schritt a) durch Einsatz folgender Primer ausgeführt wird:
i) Einem Paar Primer von SEQ.ID.NO.3 und SEQ.ID.NO.4, jeweils bezogen auf die upstream- und downstream-Primer für den LO1-Nachweis;
ii) Ein Paar Primer von SEQ.ID.NO.7 und SEQ.ID.NO. 8 sind jeweils die upstream- und downstream-Primer für den L02-Nachweis;

23. Die Methode gemäß der Patentansprüche 21-22, wobei das Paar der Primer i) und ii) zwei interne Fragmente des RHD-Virus-Genoms erweitert und jedes eine der zwei Nichtübereinstimmungen zwischen der LO1-Genom-Sequenz und der LO2-Genom-Sequenz jeweils in Position 2115 und 2662 beherbergt.

## Revendications

1. Un vaccin contre le virus de la maladie hémorragique du lapin qui comprend une quantité immunologiquement efficace de souches du virus de la maladie hémorragique du lapin déposées à la *European Collection of Cell Cultures (ECACC),* CEPR, Porton Down, Salisbury, Wiltshire, SP4 0JG, GB, sous les numéros RHDV-LO1 07060601 et RHDV-L02 07060602, optionnellement ensemble avec un vecteur physiologiquement acceptable.

2. Le vaccin conformément à la revendication 1 qui inclut un adjuvant sélectionné entre l'hydroxyde d'aluminium, une huile minérale incomplète et leurs mélanges.

3. Le vaccin conformément à l'une des revendications 1 et 2, **caractérisé par le fait que** les deux souches de virus de la maladie hémorragique du lapin sont chimiquement inactivées.

4. Le vaccin conformément à la revendication 3, où l'inactivation chimique est effectuée par traitement du virus avec un agent chimique d'inactivation sélectionné entre le [bêta]-propiolactone et le formaldéhyde.

5. Le vaccin conformément à l'une des revendications 1 à 4, **caractérisé par le fait que** le taux de mélange pour chacun des virus du vaccin était de 128 HA/dose ou supérieur pour la souche de virus RHD LO1 07060601 et de 128 HA/dose ou supérieur pour la souche de virus RHD LO2 07060602.

6. Le vaccin conformément à l'une des revendications 1 à 5, **caractérisé par le fait que** les virus sont présents dans les vaccins à un taux compris entre 1:10 et 10:1.

7. Le vaccin conformément à l'une des revendications 1 à 6, **caractérisé par le fait que** les virus sont présents dans les vaccins à un taux compris entre 1:6 et 6:1.

8. Le vaccin conformément à l'une des revendications 1 à 7, **caractérisé par le fait que** les virus sont présents dans les vaccins à un taux de 1:1.

9. Le vaccin conformément à l'une des revendications 1 à 8, **caractérisé par le fait qu'**il comprend un mélange substantiellement homogène des souches du virus de la maladie hémorragique du lapin RHDV-LO1 07060601 et RHDV-L02 07060602 qui n'est contaminé par plus de 10 % d'un autre isolat du virus de la maladie hémorragique du lapin.

10. Le vaccin conformément à l'une des revendications 1 à 9, **caractérisé par le fait qu'**il comprend un mélange homogène pur des souches du virus de la maladie hémorragique du lapin RHDV-LO1 07060601 et RHDV-L02 07060602.

11. Le vaccin conformément à l'une des revendications 1 à 10, **caractérisé par le fait qu'**il contient un dosage viral efficace dans la plage de 128 à 512 HA/dose.

12. L'utilisation d'une souche du virus de la maladie hémorragique du lapin possédant la séquence des nucléotides décrite dans la SEQ.ID.NO.1 dans la préparation d'un vaccin d'immunisation contre une infection virale causée par le virus de la maladie hémorragique du lapin.

13. L'utilisation d'une souche du virus de la maladie hémorragique du lapin possédant la séquence des nucléotides décrite dans la SEQ.ID.NO.2 dans la préparation d'un vaccin d'immunisation contre une infection virale causée par le virus de la maladie hémorragique du lapin.

14. L'utilisation d'une souche du virus de la maladie hémorragique du lapin inactivée possédant la séquence des nucléotides décrite dans la SEQ.ID.NO.1 et d'une souche du virus de la maladie hémorragique du lapin inactivée ayant la séquence des nucléotides décrite dans la SEQ.ID.NO.2, dans la fabrication d'un médicament destiné à induire l'immunité chez un mammifère susceptible d'infection par le virus de la maladie hémorragique du lapin.

15. L'utilisation d'un mélange homogène pur immunologiquement efficace de la souche du virus de la maladie hémorragique du lapin possédant la séquence des nucléotides décrite dans la SEQ.ID.NO.1 et de la souche du virus de la maladie hémorragique du lapin inactivée ayant la séquence des nucléotides décrite dans la SEQ.ID.NO.2, dans la fabrication d'un vaccin destiné à immuniser contre une infection virale causée par le virus de la maladie hémorragique du lapin.

16. Une méthode de préparation du vaccin conformément à l'une des revendications 1 à 11, qui comprend :
a) mélange d'une quantité immunologiquement efficace des souches du virus de la maladie hémorragique du lapin déposées à la *European Collection of Cell Cultures (ECACC),* CEPR, Porton Down, Salisbury, Wiltshire, SP4 0JG, GB, sous les numéros RHDV-LO1 07060601 et RHDV-L02 07060602 ;
b) optionnellement en inactivant chimiquement les souches du virus ;
c) adjuvantation du produit final.

17. La méthode conformément à la revendication 16, qui comprend l'inactivation chimique des souches du virus.

18. La méthode conformément à la revendication 17, où l'inactivation chimique est effectuée par traitement du virus avec un agent chimique d'inactivation sélectionné entre le [bêta]-propiolactone et le formaldéhyde.

19. Une souche du virus de la maladie hémorragique du lapin qui est déposée à la *European Collection of Cell Cultures (ECACC),* CEPR, Porton Down, Salisbury, Wiltshire, SP4 0JG, GB, sous le numéro RHDV-LO1 07060601.

20. Une souche du virus de la maladie hémorragique du lapin qui est déposée à la *European Collection of Cell Cultures (ECACC),* CEPR, Porton Down, Salisbury, Wiltshire, SP4 0JG, GB, sous le numéro RHDV-L02 07060602.

21. Un méthode de détection et de différentiation de souches du virus de la RHD atténuées dans un vaccin possédant une séquence de nucléotides décrite dans la SEQ.ID.NO.1 et une séquence de nucléotides décrite dans la SEQ.ID.NO.2, qui comprend :
a) soumettre l'ADN génomique d'un échantillon de vaccin contre la RHD à une analyse de séquences par RT-PCR et confirmer que l'ADN génomique du dit échantillon du vaccin contre la RHD contient un produit de PCR spécifique de 359 pb et de 371 pb ;
b) faire digérer le produit de PCR avec les enzymes de restriction Mval et Ec113611 ;
c) confirmer par analyse de polymorphie de longueur de fragment de restriction la présence d'une séquence de reconnaissance de Mval à la position 2662 mais sans séquence de reconnaissance de Ec113611 à la position 2115 pour la souche de virus de RHD LO1 possédant une séquence de nucléotides décrite dans la SEQ.ID.NO.1, et une séquence de reconnaissance de Ec113611 à la position 2115 mais sans séquence de reconnaissance de Mval à la position 2662 pour la souche de virus de RHD LO2 possédant une séquence de nucléotides décrite dans la SEQ.ID.NO.2.

22. La méthode conformément à la revendication 21 où l'étape de RT-PCR a) est réalisée en utilisant les amorces suivantes :
i) une paire d'amorces de SEQ.ID.NO.3 et de SEQ.ID.NO.4 liées aux amorces en amont et en aval, respectivement, pour la détection de LO1 ;
ii) une paire d'amorces de SEQ.ID,NO.7 et de SEQ.ID.NO.8 liées aux amorces en amont et en aval, respectivement, pour la détection de L02.

23. La méthode conformément à l'une des revendications 21 et 22, où la paire d'amorces i) et ii) amplifient deux fragments internes du génome du virus de la RHD, hébergeant chacun l'un des deux mésappariement entre la séquence du génome de LO1 et la séquence du génome de LO2 aux positions 2115 et 2662, respectivement.


### SEQUENCE LISTING

<110> Laboratorios Ovejero
   <120> Vaccine against viral rabbit hemorrhagic disease
   <130> 0071-2006
   <160> 10
   <170> Patentln version 3.3
<210> 1
   <211> 4802
   <212> DNA
   <213> Rabbit hemorrhagic disease virus RHDV-LO1 0706060
   <400> 1
   tactggcatg actactgcta ttttaccaga gaagaaaccc ttaaatttct tcctggacct 60
   cagggacaag acccctccct gttgtattcg ggcaaccggg aaactgtctt ggccagtctt 120
   tctgggacaa aacggaagag aaggacctct ggagacatgt aacaaatgtg gcaaatggct 180
   taacgaattt ggctgttttg gattagagga tcttggtgat gtttgcttgt gttccatagt 240
   ccaacagaaa cacaagtttg gacctgtctg cctgtgcaat cgtgcttaca tacacgactg 300
   cggtcgttgg cggcgccgca gtcgtttctt gaagcactac aaggcattga acaaggttat 360
   cccctgtgct taccagtttg acgagagcct ctccacccct gtttttgagg gggaggttga 420
   cgacttgttt gttgagcttg gagcaccaac tagcatgggt tttatagaca agaagttgct 480
   caaaaagggt gagaagctta tggacaagtt tgttgacgtt gacgaaccct gtttgacatc 540
   aagagacgcc agcttactcg actccatagc ttcggacaac accatacgtg cgaagctgga 600
   agaagagtat ggtgttgaaa tggtgcaggc agcgcgtgac aggaaagact tcatgaagaa 660
   tctcaggctc gccttggaca accggcccgc gaaccctgtt acctggtaca ctaaacttgg 720
   caacataacc gaaaaaggga agcagtgggc gaagaaggta gtttatggtg cctgcaaggt 780
   gactgacccc ctgaaaacgt tggcttcaat cttgctagtt ggtctccaca atgtcatcgc 840
   cgtggacaca acagtcatgt tgtcaacgtt taaacccgtg aacctgctag cgatcctcat 900
   ggactggacc aacgacttga ctgggtttgt taccacacta gtgaggctgt tggagctgta 960
   tggtgtggtg caggcaactg ttaacctgat cgttgagggt gtgaagagtt tctgggacaa 1020
   ggtagtttgt gcaacggacc gctgctttga cttgctcaag aggctgtttg acacgtttga 1080
   gaactccgtc cccacgggac caaccgctgg ctgtctcatc ttcatggctt ttgtcttttc 1140
   cactgttgtt gggtacctgc ccaacaatag tgtgatcacg acattcatga agggtgctgg 1200
   caagctcaca acctttgcag gggttattgg tgccatccgg acgttgtgga tcactattaa 1260
   ccaacacatg gtggccaagg acctcaccag tatacagcaa aaggttatga cagttgtgaa 1320
   aatggctaat gaggcagcaa cgttggacca gctcgagatc gtcagctgcc tctgctcaga 1380
   cttggagaac acactaacaa ataggtgcac cctgccatca tataaccaac atcttgggat 1440
   cttaaacgca tcccagaaag ttataagtga cttacacacc atggtccttg gaaagatcaa 1500
   catgacgaag cagaggccac aacctgtggc tgtcattttc aaaggagccc caggcattgg 1560
   taaaacctac ctagtccaca ggattgcacg ggatttgggc tgccagcacc caagcacaat 1620
   caattttggc cttgaccact tcgactcgta cacaggtgag gaggttgcta ttgcggacga 1680
   gttcaacact tgtggtgacg gagagagttg ggttgaactg ttcatccaga tggtcaacac 1740
   gaacccgtgc ccactcaact gtgacaaggc tgagaacaag aacaaagttt tcaactcaaa 1800
   gtacctgctg tgcacaacaa attccaacat gatactgaat gcgacccatc ctcgtgctgg 1860
   cgcattttat agaagggtta tgattgttga agcacgtaac aaggctgttg aaagctggca 1920
   agccaccagg catggctcca aaccggggag gtcctgctac agcaaggata tgtcgcacct 1980
   gactttccag gtttaccccc acaacatgcc tgccccaggg tttgtgtttg ttggggacaa 2040
   actggtgaag tctcaggtcg ctccaaggga gtacaagtac agtgaactct tggacctcat 2100
   caagagtgag caccccgacg tggcctcatt tgagggtgcc aacagattca actttgtcta 2160
   cccagacgcc caatacgacc aggcgctcct catgtggaaa cagtattttg tcatgtatgg 2220
   ttgcgtggca aaacttgcga agaatttcgt agatgacatc ccatacaatc aggttcacat 2280
   atcgagggcg agcgacccaa agattgaagg gtgtgtggag taccattgca agttccaaca 2340
   tctctggcgc atggttcccc agtttgtttt ggggtgtgtg aacatgacaa atcagcttgg 2400
   cacccctctg acccaacagc agctagacag ggtcaccaac ggggtcgagg gagttactgt 2460
   cacaacggtg aacaacatcc tgccgttcca ctcacagacc acgctcatca acccctcttt 2520
   catcaagctg atttgggcag tcagaaaaca cctcaaagga cttagcggcg taaccaaggt 2580
   ggctcaattc atctggcgcg tcatgactaa cccagtggac gcctatggtt ccctggttag 2640
   aaccctcacc ggggctgcca ccttctcaga tgacccggtt tccacaacaa ttatctgttc 2700
   caactgtaca attcagatcc actcctgcgg gggactactt gtcaggtact caagggatcc 2760
   tgtccccgtg gcatcagaca acgttgaccg cggtgaccaa ggtgttgacg tgttcaccga 2820
   ccccaacctc atttctgggt ttagttggag gcagattgcg catctatttg ttgaggtgat 2880
   ctcacatttg tgtgcaaacc accttgtcaa ccttgcaacc atggcggccc tgggcgcagt 2940
   tgcgacgaaa gcttttcagg gcgtgaaggg caagacgaaa cgtggtcgtg gtgcgagagt 3000
   gaacttgggc aacgacgagt atgacgaatg gcaagctgca cgcagagagt tcgtcaacgc 3060
   acacgatatg actgcagagg agtatcttgc gatgaagaat aaagctgcca tgggaagtga 3120
   cgaccaagac tccgtcatgt ttaggagctg gtggacccgg agacaactga gacctgatga 3180
   ggaccaagtc accgttgttg gtaggggtgg agtgagaaac gagatcatcc gcaccagagt 3240
   tagacaaact ccaaaaggac caaagacgct agacgacggt ggcttttatg acaatgacta 3300
   tgagggcctg cctgggttca tgagacacaa tggcagtggc tggatgatcc acataggtaa 3360
   tgggttgtac atctccaaca ctcacaccgc caggtcaagt tgctcagaag ttgtcacatg 3420
   ctcacccacc actgacctgt gcctcgtcaa gggcgaggta atccgcagcg tcgctcagat 3480
   tgctgagggt accccagttt gcgactggaa gaagtcccca atttcgacct atggcatcaa 3540
   gaagacattg tcagactcca ccaagattga cgtgctggcg tacgacgggt gcacgcagac 3600
   cacccacggt gactgtgggc tgccgttgta tgactccagc ggaaagattg ttgccatcca 3660
   cactggcaag ttgttggggt tttcaaagat gtgcacgttg attgacctca ccatcacaaa 3720
   gggagtttat gaaacatcaa acttcttctg cggcgagcca attgactaca gaggcatcac 3780
   cgcacacagg ctcgtcggcg cggaacccag gcctcccgtt agtggaacca ggtatgctaa 3840
   ggtcccaggt gttcctgaag agtacaagac tgggtacaga ccagccaacc ttgggcgcgg 3900
   tgacccagac agtgacaagt cactcatgaa cattgcggtg aagaacttac aggtgtacca 3960
   gcaagaacca aagctcgata aggtggatga gtttatcgag cgagcggctg ccgacgtctt 4020
   gggatactta cgcttcctaa ccaaggggga gcgccaggca aatctaaact ttaaagccgc 4080
   attcaacacc ctagacttgt caacctcttg cgggcccttt gttccaggga agaagattga 4140
   tcatgtgata gatggagtga tggaccaggt cctagccaag cacctttaca agtgctggag 4200
   tgttgccaac tctggcaaag cccttcacca catatatgcg tgtgggttga aggacgagct 4260
   caggcctctt gacaaggtga aagagggtaa gaagaggttg ctgtggggtt gtgacgttgg 4320
   tgtcgccgtg tgcgctgccg ctgttttcca caacatatgc tacaaactaa aaatggttgc 4380
   acggtttggc cctattgcag ttggcgttga catgacatca cgtgacgtgg acgtcatcat 4440
   caataacctt acctccaaag ccagcgactt cttgtgcttg gactactcca agtgggactc 4500
   caccatgtca ccgtgtgttg tcaggctagc cattgacatc ctagccgatt gttgtgagca 4560
   aacggagctc accaaaagtg tcgtgctcac actgaagtcc caccccatga ccatacttga 4620
   cgctatgatt gtgcaaacca agcgtggcct tccgagtggc atgcccttca cctcggtcat 4680
   caactccata tgtcattggt tgctttggtc tgcagccgtt tacaagtctt gtgcagaaat 4740
   tggcttgcat tgctccaacc tgtacgagga cgcccctttc tacacgtatg gtgatgatgg 4800
   cg 4802
<210> 2
   <211> 4802
   <212> DNA
   <213> Rabbit hemorrhagic disease virus RHDV-LO2 07060602
   <400> 2
   tactggcatg actactgcta ttttaccaga aaagaaaccc ttgaatttct tcctggacct 60
   cagggacaag acccctccct gttgtattcg ggcaaccggg aaactggctt ggccagtctt 120
   tctgggacaa aacggaaaag aaggacctct agagacatgt gacaaatgtg gcaaatggct 180
   taacggattt ggttgttttg gattagagga tcttggtgat gtttgcttgt gttccatagc 240
   ccaacagaaa cacaagtttg gacctgtctg cctgtgcaat cgtgcttaca tacacgactg 300
   cggtcgttgg cggcgccgca gtcgtttctt gaagcactac aaggcattga acaaggttat 360
   cccctgtgct taccagtttg acgaaatttt ctccacccct gtttttgagg gggaggttga 420
   cgacttgttt gttgagcttg gagcaccaac tagcatgggc tttatggata agaagttgct 480
   caaaaagggt aagaagctta tggataagtt tgttgacgtt gacgaacctt gtttaacatc 540
   aagagacgcc agcctacttg actccatagc ttcggacaac accatacgtg cgaagctgga 600
   agaggagtat ggtgttgaaa tggtgcaagc agcgcgtgac aggaaagact tcatgaagaa 660
   cctcaggctc gccttggaca accggcccgc gaaccctgtt acctggtaca ctaaacttgg 720
   caacataaca gaaaaaggga agcagtgggc aaagaaggtt gtttatggtg cctgcaaggt 780
   gactgacccc ctgaaaacgt tggcttcaat cttgctagtt ggcctccaca atgtcatcgc 840
   cgtggacaca acagtcatgt tgtcaacgtt caaacccgtg aacctgctag cgatcctcat 900
   ggactggacc aatgacttaa ctgggtttgt taccacacta gtgaggctgt tggaactgta 960
   tggtgtggtg caggcaactg tcaacctgat cgttgagggt gtgaagagtt tctgggacaa 1020
   ggtggtttgt gcaacggacc gctgctttga cttgctcaag aggctgtttg acacgtttga 1080
   ggactccgtc cccacgggac caaccgctgg ctgtctcatc ttcatggctt ttgtcttttc 1140
   cactgttgtt gggtacctgc ccaacaacag tgtgatcacg acattcatga agggtgctgg 1200
   caagctcaca acctttgcag gagttattgg tgccatccgg acgttgtgga tcactatcaa 1260
   ccaacacatg gtggccaagg acctcaccag tatacagcaa aaggttatga cagttgtgaa 1320
   aatggctaat gaggcagcaa cgttggacca gctcgagatc gtcagctgcc tctgctcaga 1380
   cttggagaac acactaacaa ataggtgcac cctgccatca tacaaccaac atcttgggat 1440
   cttaaatgca tcccagaaag ttataagtga cttacacacc atggtccttg ggaagattaa 1500
   catgacgaag cagagaccac aacccgtggc tgtcattttc aaaggagccc caggcattgg 1560
   taaaacctac ttagtccaca ggattgcacg ggacttgggc tgccagcacc caagtacaat 1620
   caattttggc cttgaccact tcgactcgta cacaggtgag gaggttgcta ttgcggacga 1680
   gttcaacact tgtggtgacg gagagagttg ggttgaactg ttcatccaga tggtcaacac 1740
   gaacccgtgc ccactcaact gtgacaaggc tgagaacaag aacaaagttt tcaactcaaa 1800
   gtacctgctg tgcacaacaa attccaacat gatactgaat gcgacccacc ctcgtgctgg 1860
   cgcattttat agaagggtta tgattgttga agcacgcaac aaggctgttg aaagctggca 1920
   agccaccagg catggctcca aaccggggag atcctgctac agcaaggata tgtcgcacct 1980
   gactttccag gtttaccccc acaacatgcc tgccccaggg tttgtatttg ttggggacaa 2040
   actggtgaag tctcaagtcg ctccaaggga gtacaagtac agtgagctct tggaccttat 2100
   caagagtgag caccccgatg tggcctcatt tgagggtgcc aacaggttta actttgtcta 2160
   cccagacgcc caatacgacc aggcgctcct catgtggaaa cagtactttg tcatgtatgg 2220
   ttgtgtggcg agacttgcga agaatttcgt agatgacatc ccatacaatc aagttcacat 2280
   atcgagggcg agcgacccaa agattgaagg gtgtgtggag tatcagtgca agttccaaca 2340
   tctttggcgc atggttcccc agtttgtttt ggggtgtgtg aacatgacaa atcagcttgg 2400
   cacccctttg acccaacagc agctagacag gattaccaat ggggtcgagg gagtcactgt 2460
   cacaacggtt aacaacatcc tgccgttcca ctcacagacc acgctcatca acccctcttt 2520
   catcaaactg atttgggcag ttagaaaaca cctcaaagga cttagcggcg tgaccaaggt 2580
   ggcccaattc gtctggcgcg tcatgaccaa cccagtggac gcctatggat ccttggtcag 2640
   aaccctcacc ggggctgcca ccttctcaga cgacccggtt tccacaacaa ttatctgctc 2700
   caactgtaca attcagatcc actcctgcgg gggactactt gtcaggtact caagggaccc 2760
   tgtccccgtg gcatcagaca acgttgaccg cggtgaccaa ggtgttgacg tgttcaccga 2820
   ccccaacctc atttctgggt ttagctggag gcagattgcg catctatttg ttgaggtgat 2880
   ctcacacttg tgtgcaaacc accttgtcaa ccttgcaacc atggcggccc tgggcgcggt 2940
   tgcgacgaaa gcttttcagg gcgtgaaagg caagacgaaa cgtggtcgtg gtgcaagagt 3000
   gaacttgggc aatgacgagt atgacgaatg gcaagctgca cgcagagagt tcgtcaacgc 3060
   acacgatatg actgcagagg agtatctcgc gatgaagaac aaagctgcca tgggaagtga 3120
   cgaccaagac tccgtcatgt ttagaagctg gtggacccgg agacaactga gacctgatga 3180
   ggaccaagtt accgttgttg gtaggggtgg agtgagaaac gaggtcatcc gcaccagggt 3240
   tagacaaact ccaaaagggc caaagacact agacgacggt ggcttttatg acaatgacta 3300
   tgagggcctg cctgggttca tgagacacaa tggcagtggc tggatgatcc acataggtaa 3360
   cgggttgtac atctccaaca ctcacaccgc caggtcaagt tgctcagaag ttgtcacatg 3420
   ctcacccacc actgacctgt gcctcgtcaa gggtgaggca atccgcagtg ttgctcagat 3480
   tgctgagggt acccagatct gcgactggaa gaagtcccca atttcgacct atggcatcaa 3540
   gaagacattg tcagactcca ccaagattga cgtgctggcg tacgacgggt gcacgcagac 3600
   cacccacggt gactgtgggc tgccgttgta tgactccagc ggaaagattg ttgccatcca 3660
   cactggcaag ttgttggggt tttcaaagat gtgcacgttg attgatctca ccatcacaaa 3720
   gggagtttat gaaacatcaa acttcttctg cggcgagcca attgactata gaggcatcac 3780
   cgcacacagg ctcgtcggcg cggaacccag acctcccgtt agtggaacca ggtatgctaa 3840
   ggtcccaggt gttcctgaag agtataagac tgggtacaga ccagccaacc ttgggcgcgg 3900
   tgacccagac agtgacaagt cactcatgaa cattgcggtg aaaaacttgc aggtgtacca 3960
   gcaagaacca aagctcgata aggtggatga gtttatcgag cgagcggctg ccgacgtctt 4020
   gggatactta cgcttcctaa ccaaggggga gcgccaggca aacctaaact ttaaagccgc 4080
   attcaatacc ctagacctgt caacctcttg cgggcccttt gttccaggga agaagattga 4140
   tcatgtgaaa gatggagtga tggaccaagt cctagccaag cacctttaca agtgctggag 4200
   tgttgccaac tctggcaaag cccttcacca catatacgcg tgtgggttga aggacgagct 4260
   caggcctctt gacaaggtga aagagggaaa gaagaggttg ctatggggtt gtgacgttgg 4320
   tgtcgccgtg tgcgctgccg ctgtttttca caacatatgc tacaagctaa aaatggttgc 4380
   acgatttggc cctattgcgg ttggcgttga catgacatcc cgtgacgtgg acgtcatcat 4440
   caacaaccta acctccaaag ccagcgactt cttgtgcttg gattactcaa agtgggactc 4500
   caccatgtca ccgtgtgttg tcaggctagc cattgacatc ttagccgatt gttgtgagca 4560
   aacggagctc accaaaagtg tcgtgctcac actgaaatcc caccccatga ccatacttga 4620
   cgccatgatt gtgcaaacca agcgtggtct tccgagtggc atgcccttca cctcggtcat 4680
   caattccatt tgccattggt tgctttggtc tgcagccgtt tacaagtctt gtgctgaaat 4740
   tggcttacat tgctccaacc tgtacgagga cgcccctttc tacacgtatg gtgatgacgg 4800
   cg 4802
<210> 3
   <211> 21
   <212> DNA
   <213> Artificial
   <220>
   <223> upstream primer of the PCR RHD-QC-1
   <400> 3
   aacaacatcc tgccgttcca c 21
<210> 4
   <211> 22
   <212> DNA
   <213> Artificial
   <220>
   <223> Downstream primer of the PCR RHD-QC-1
   <400> 4
   aggttggggt cggtgaacac gt 22
<210> 5
   <211> 359
   <212> DNA
   <213> Artificial
   <220>
   <223> Sequence of the PCR amplicon of PCR RHD-QC-1 of RHD virus strain L01
   <400> 5
   aacaacatcc tgccgttcca ctcacagacc acgctcatca acccctcttt catcaagctg 60
   atttgggcag tcagaaaaca cctcaaagga cttagcggcg taaccaaggt ggctcaattc 120
   atctggcgcg tcatgactaa cccagtggac gcctatggtt ccctggttag aaccctcacc 180
   ggggctgcca ccttctcaga tgacccggtt tccacaacaa ttatctgttc caactgtaca 240
   attcagatcc actcctgcgg gggactactt gtcaggtact caagggatcc tgtccccgtg 300
   gcatcagaca acgttgaccg cggtgaccaa ggtgttgacg tgttcaccga ccccaacct 359
<210> 6
   <211> 289
   <212> DNA
   <213> Artificial
   <220>
   <223> Sequence of the PCR amplicon of PCR RHD-QC-1 of RHD virus strain LO2
   <400> 6
   ttagaaaaca cctcaaagga cttagcggcg tgaccaaggt ggcccaattc gtctggcgcg 60
   tcatgaccaa cccagtggac gcctatggat ccttggtcag aaccctcacc ggggctgcca 120
   ccttctcaga cgacccggtt tccacaacaa ttatctgctc caactgtaca attcagatcc 180
   actcctgcgg gggactactt gtcaggtact caagggaccc tgtccccgtg gcatcagaca 240
   acgttgaccg cggtgaccaa ggtgttgacg tgttcaccga ccccaacct 289
<210> 7
   <211> 21
   <212> DNA
   <213> Artificial
   <220>
   <223> upstream primer of the PCR RHD-QC-2
   <400> 7
   tcctgctaca gcaaggatat g 21
<210> 8
   <211> 21
   <212> DNA
   <213> Artificial
   <220>
   <223> downstream primer of the PCR RHD-QC-2
   <400> 8
   tactccacac acccttcaat c 21
<210> 9
   <211> 371
   <212> DNA
   <213> Artificial
   <220>
   <223> Sequence of the PCR amplicon of PCR RHD-QC-2 of RHD virus strain L01
   <400> 9
   tcctgctaca gcaaggatat gtcgcacctg actttccagg tttaccccca caacatgcct 60
   gccccagggt ttgtgtttgt tggggacaaa ctggtgaagt ctcaggtcgc tccaagggag 120
   tacaagtaca gtgaactctt ggacctcatc aagagtgagc accccgacgt ggcctcattt 180
   gagggtgcca acagattcaa ctttgtctac ccagacgccc aatacgacca ggcgctcctc 240
   atgtggaaac agtattttgt catgtatggt tgcgtggcaa aacttgcgaa gaatttcgta 300
   gatgacatcc catacaatca ggttcacata tcgagggcga gcgacccaaa gattgaaggg 360
   tgtgtggagt a 371
<210> 10
   <211> 371
   <212> DNA
   <213> Artificial
   <220>
   <223> Sequence of the PCR amplicon of PCR RHD-QC-2 of RHD virus strain LO2
   <400> 10
   tcctgctaca gcaaggatat gtcgcacctg actttccagg tttaccccca caacatgcct 60
   gccccagggt ttgtatttgt tggggacaaa ctggtgaagt ctcaagtcgc tccaagggag 120
   tacaagtaca gtgagctctt ggaccttatc aagagtgagc accccgatgt ggcctcattt 180
   gagggtgcca acaggtttaa ctttgtctac ccagacgccc aatacgacca ggcgctcctc 240
   atgtggaaac agtactttgt catgtatggt tgtgtggcga gacttgcgaa gaatttcgta 300
   gatgacatcc catacaatca agttcacata tcgagggcga gcgacccaaa gattgaaggg 360
   tgtgtggagt a 371